Europäisches Patentamt

European Patent Office

Office européen des brevets

(19)

(11) Veröffentlichungsnummer : **0 126 978**
**B1**

(12)

# EUROPÄISCHE PATENTSCHRIFT

(45) Veröffentlichungstag der Patentschrift :
10.09.86

(51) Int. Cl.⁴ : **A 61 F   2/38**

(21) Anmeldenummer : 84104649.3

(22) Anmeldetag : 25.04.84

(54) Kniegelenkendoprothese.

(30) Priorität : 28.04.83 DE 3315401

(43) Veröffentlichungstag der Anmeldung :
05.12.84 Patentblatt 84/49

(45) Bekanntmachung des Hinweises auf die Patenterteilung : 10.09.86 Patentblatt 86/37

(84) Benannte Vertragsstaaten :
AT BE CH DE FR GB IT LI LU NL SE

(56) Entgegenhaltungen :
DE-A- 2 549 819
DE-A- 2 906 458
DE-A- 2 914 737
DE-A- 3 039 992
GB-A- 2 120 943
US-A- 4 094 017
US-A- 4 215 439
US-A- 4 216 549
US-A- 4 340 978

(73) Patentinhaber : Feldmühle Aktiengesellschaft
Fritz-Vomfelde-Platz 4
D-4000 Düsseldorf 11 (DE)

(72) Erfinder : Zichner, Ludwig, Dr.
Auf dem Mühlberg 8
D-6000 Frankfurt (DE)
Erfinder : Dörre, Erhard, Dr.
Talweg 14
D-7310 Plochingen (DE)
Erfinder : Prüssner, Peter, Ing. grad.
Waldstrasse 38
D-6057 Dietzenbach (DE)
Erfinder : von Borries, Horst
An der Lunie 2
D-4150 Krefeld 29 (DE)

(74) Vertreter : Uhlmann, Hans, Dr. rer.nat., Dipl.-Chem.
Gladbacher Strasse 189
D-4060 Viersen 1 (DE)

EP 0 126 978 B1

**Beschreibung**

Die Erfindung betrifft eine Kniegelenkendoprothese zur Verbindung des Femur mit der Tibia, bestehend aus einer Femurkappe mit hakenförmigem Querschnitt zur teilweisen Umfassung der präparierten Femur-Condylen und gleichzeitiger Abstützung und Lagerung auf dem Tibia-Plateau sowie einem in die Tibia implantierbaren Verbindungsstück.

Kniegelenkendoprothesen, die das gesamte Gelenk ersetzen, also eine Resektion des Gesamtgelenkbereiches erfordern, sind beispielsweise aus der DE-A-29 06 458 und der DE-A-25 49 819 bekannt. Ziel einer Prothesenkonstruktion ist jedoch, nach Möglichkeit nur die Gleitflächen zu ersetzen und dennoch, auch bei Bandinstabilität, eine feste Führung der knöchernen Gelenkpartien zu erreichen.

Es ist deshalb bereits mit der DE-A-30 39 992 vorgeschlagen worden, den condylaren Flächenbereich so zu resezieren, daß mit Knochenzement eine Femurkappe darauf befestigt werden kann. Diese Femurkappe umgreift das periphere Ende des resezierten Femur und weist als äußeren Mantel zwei sphärische Bereiche entsprechend den Condylen auf. Diese beiden sphärischen Bereiche stehen dabei mit einer Pfanne als Ersatz des Tibia-Plateaus in Verbindung, bei der ein Dornansatz die beiden kugelkalottenförmigen Pfannenbereiche verbindet. Die Pfanne wird ebenfalls mit Knochenzement eingesetzt, d. h., in das Schienbein eingekittet. Beim Aushärten von Knochenzementen entsteht jedoch stets eine relativ hohe Temperatur, so daß eine vorübergehende Schädigung des Knochengewebes eintritt. Gegen die Anwendung von Zement sprechen auch Antikörperreaktionen und biomechanische Faktoren, insbes. beim jüngeren Patienten. Man versucht daher, nach Möglichkeit eine Verankerung mit Knochenzement zu vermeiden.

Aufgabe der vorliegenden Erfindung ist es, ein implantierbares Gelenk zu schaffen, das diese Nachteile nicht aufweist und unter Beibehaltung der physiologischen Bewegungsmöglichkeit des Gelenkes, nämlich Beugen des Beines, auch bei geschädigten Bändern, eine sichere Verbindung zwischen dem Ober- und Unterschenkelknochen zu gewährleisten. Desweiteren soll die Implantation möglichst wenig Knochenresektion erforderlich machen, um die Möglichkeit einer Nachoperation offen zu lassen. Besondere Bedeutung kommt jedoch dabei der sogenannten Primärfixation zu. Bei mit Knochenzement eingebrachten Implantaten kann man normalerweise keinen Unterschied zwischen Primär- und Sekundärfixation feststellen, da die absolute Verankerung durch den Knochenzement erfolgt. Nach der Aushärtung des Zementes ist also das Gelenk bereits belastbar. Wird eine andere Befestigungsmöglichkeit für die einzelnen Gelenkprothesen gewählt — beispielsweise Verschrauben oder Nageln — so erreicht das Gelenk nicht sofort seine volle Belastbarkeit. Es ist zwar im Knochen fixiert, d. h. primärfixiert, die endgültige Verankerung erfolgt jedoch erst mit dem Einwachsen des Knochens ; die Endoprothese muß erst einheilen. Dieser Einheilprozeß dauert umso länger, je schwächer die Primärfixation ist, weil jede Bewegung zwischen Knochen und Implantat den Einwachsprozeß stört. Schwache Primärfixationen erfordern daher eine Ruhigstellung der Gliedmaßen, was auf der einen Seite eine erhebliche Behinderung für den Patienten darstellt, auf der anderen Seite aber auch die Gefahr der Bewegungsbeeinträchtigung der Gliedmaßen mit sich bringt, wenn die Einheilperiode zu lange dauert. Ebenso treten Thrombosen und Embolien dann häufiger auf.

Eine bevorzugte Aufgabe der Erfindung ist deshalb die Schaffung einer Kniegelenkendoprothese, die eine sehr gute Primärfixation aufweist, dadurch die Einheilungsphase verkürzt und eine baldige Belastung der Prothese ermöglicht.

Diese Aufgabe wird gelöst durch eine Kniegelenkendoprothese zur Verbindung des Femur mit der Tibia, bestehend aus einer Femurkappe mit hakenförmigen Querschnitt zur teilweisen Umfassung der resezierten Femur-Condylen und gleichzeitiger Abstützung und Lagerung auf dem Tibia-Plateau sowie einem in die Tibia implantierbaren Verbindungsstück, und ist durch die Kombination folgender Merkmale gekennzeichnet :

a) der Querschnitt der Femurkappe ist C-förmig,

b) der C-förmige Querschnitt der Femurkappe ist durch zwei unterschiedlich große Bögen gebildet, die durch eine Linie miteinander verbunden sind,

c) im Bereich des großen Bogens der Femurkappe verläuft radial ein Langloch, durch das sich ein in die Tibia implantierbarer Schaft erstreckt.

Der C-förmige Querschnitt der Femurkappe mit seinen beiden Bögen gestattet eine feste Verankerung auf den resezierten Femur-Condylen ohne Knochenzement. Die Primärfixation ergibt sich durch eine Passung zwischen der Innenfläche der Femurkappe und der Außenfläche der resezierten Femur-Condylen. Durch diese Passung alleine wäre jedoch noch eine Verschiebung der Femurkappe in der Laufrichtung der Achse beider Bögen möglich. Die Arretierung dieser Bewegungsmöglichkeit erfolgt durch Nageln oder Verschrauben der Femurkappe mit dem Femur oder, gemäß einer bevorzugten Ausgestaltung der Erfindung, durch Eingriff des Schaftes in einen im Bereich des großen Bogens drehbar angeordneten, sich beidseitig über das Langloch erstreckenden Zylinder, der praktisch spielfrei zwischen den beiden resezierten Femur-Condylen eingesetzt wird und eine Bohrung zur Aufnahme des Schaftes aufweist. Dieser Schaft wird in der Tibia verankert. Er erstreckt sich durch das Langloch im Bereich des großen Bogens der Femurkappe, wodurch eine Bewegung der Femurkappe in Laufrichtung der Achse des großen Bogens verhindert wird.

Damit ist in geradezu idealer Weise eine Primärfixation der Femurkappe ohne Zuhilfenahme von Knochenzement erreicht, die zudem noch den großen Vorteil aufweist, daß der Knochen nur minimal reseziert werden muß.

Die äußere Gestalt der Femurkappe entspricht im wesentlichen der natürlichen Gelenkform und ist gemäß seiner vorteilhaften Ausgestaltung in der Erfindung poliert.

Die Tibia ist gleich zweifach gelagert. Sie stützt sich zum einen — bei Druckbelastung — auf dem Umfang der Femurkappe ab, wobei auch hier ein Implantat als Ersatz des Tibia-Plateaus eingesetzt wird, zum anderen ist die Tibia über den Schaft mit dem Zylinder verbunden, so daß bei Zugbeanspruchung die Gleitbewegung in den großen Bogen der Femurkappe, also auf Zylinder und Innenbereich der Femurkappe verlagert wird. Eine vorteilhafte Ausgestaltung der Erfindung sieht deshalb vor, daß die Femurkappe in dem an das Langloch angrenzenden Innenbereich des großen Bogens poliert ist.

Um die Reibung weiter zu reduzieren, sind zweckmäßig auch die Stirnflächen des Zylinders poliert, die bei der Bewegung des Knies an den resezierten Seiten der Femur-Condylen gleiten. Ebenso vorteilhaft ist die Mantelfläche des Zylinders poliert, um eine möglichst geringe Reibung zwischen Zylinder und Femurkappe zu erreichen.

Als Material für die Endoprothese können praktisch alle biokompatiblen Werkstoffe eingesetzt werden, die die nötige Festigkeit aufweisen. Es muß jedoch darauf geachtet werden, daß sie beim Zusammenarbeiten keinen Abrieb verursachen. So sind beispielsweise Kombinationen von Femurkappe aus Oxidkeramik oder Metall mit einem Zylinder aus HD-Polyäthylen möglich. Nicht verwendbar sind jedoch Keramik/Metall-Paarungen oder Metall/Metall-Paarungen, bei denen die Gefahr des Metallabriebes auf Grund der Bewegung besteht. Wegen der extrem niedrigen Abriebwerte, die praktisch bei nahe Null liegen und der ausgezeichneten Körperverträglichkeit, wird vorzugsweise sowohl für die Femurkappe als auch für den Zylinder Oxidkeramik eingesetzt. Der Begriff Oxidkeramik wird genauer als hochreine Sinteroxidkeramik definiert, wobei unter dem Begriff « hochrein » zu verstehen ist, daß die Sinteroxidkeramik eine Reinheit von über 95 % aufweist, und unter dem Begriff Sinteroxidkeramik, im Sinne der vorstehenden Ausführungen, die gesinterten Metalloxide von Zirkon, Titan und insbesondere Aluminium, sowie deren Gemische zu verstehen sind.

Bevorzugt wird gesintertes Aluminiumoxid mit einer Dichte = oder > $3,92$ g/cm$^2$, einer Porosität = oder < 2 %, einer Wasseraufnahme = oder < 0,01 %, einer Reinheit = oder > 99,7 % $Al_2O_3$, einer Vickershärte (P = 2 M) = oder > 22 000 N/mm$^2$, einer mittleren Korngröße = oder < 10 $\mu$m, einer mittleren Biegefestigkeit = oder > 320 N/mm$^2$, einer Druckfestigkeit = oder > 4 000 N/mm$^2$, und einer Zugfestigkeit = oder > 160 N/mm$^2$ eingesetzt.

Ein derartiges Material ergibt die erforderliche hohe Sicherheit für eine Endoprothese über Jahrzehnte hinaus, wobei gerade durch die Kombination der hohen Dichte mit der niedrigen mittleren Korngröße und der hohen Reinheit des gesinterten Aluminiumoxides die hervorragenden Eingenschaften erreicht werden. Unter Reinheit ist dabei zu verstehen, daß in dem Aluminiumoxid möglichst wenig Fremdstoffe als weitere Bestandteile enthalten sein sollen, die zu einer glasartigen Zwischen- oder Übergangsphase führen könnten. Nicht im Widerspruch zu dieser Forderung steht, daß dem Ausgangspulver, also dem Aluminiumoxid bestimmte Zusätze, wie beispielsweise Magnesiumoxid als Kornwachstumshemmer zugesetzt werden können.

Ein solches Material ermöglicht eine Politur, die zu sehr hohen Glättewerten führt und damit zu außerordentlich niedriger Reibung, wie sie bei einer Endoprothese erforderlich ist, d. h., daß sowohl die Reibung zwischen dem Zylinder und dem Innenbereich der Femurkappe sehr gering ist als auch die Reibung zwischen den Stirnflächen des Zylinders und den resezierten Condylenbereichen, an denen der Zylinder anliegt.

Wichtig für die Primärfixation der Femurkappe ist, daß die Maße des resezierten Condylenbereiches exakt mit der Innenbemaßung der Femurkappe übereinstimmen, so daß diese mittels Preßsitz mit dem Femur verbunden werden kann. Durch die Preßsitzverbindung ist die Verwendung von Knochenzement nicht erforderlich. Gleichzeitig wird aber sofort eine ausgezeichnete Primärfixation erreicht. Voraussetzung dafür ist jedoch, daß enge Toleranzen bei der Implantation und auch bei der Fertigung der Femurkappe eingehalten werden. Die Resektion erfolgt daher zweckmäßig mittels eines schablonengeführten Finger- oder Walzenfräsers, der entsprechend den inneren Konturen der Femurkappe geführt wird. In einem weiteren Arbeitsgang werden dann die Stirnflächen der Condylen vertikal reseziert, wobei diese beiden, sich gegenüberliegenden Flächen im eingebauten Zustand des Gelenkes die Anlage für die Stirnflächen des Zylinders bilden.

Der Zylinder ist im Bereich des großen Bogens der Femurkappe gelagert, wobei dieser große Bogen gemäß einer bevorzugten Ausgestaltung der Erfindung einen Winkel $\alpha$ von 130 bis 190 Grad umfaßt. Dieser große Bogenbereich sorgt also auf der einen Seite für eine gute Lagerung des Zylinders in der Femurkappe und ermöglicht auf der anderen Seite eine kräftige Verankerung der Femurkappe am Femur.

Der Winkel des kleineren Bogens, der gemäß einer bevorzugten Ausgestaltung der Erfindung zwischen 120 und 160 Grad liegt, umgreift im Normalfall nur einen kleinen Teilbereich der Facies Patellaris, kann aber für den Fall, daß auch hier eine größere Resektion erforderlich war, höher hinaufgezogen werden und damit die Facies Patellaris und ggf. auch die Patella komplett ersetzen.

Gemäß vorteilhaften Ausgestaltungen der Erfindung beträgt der Innenradius RB des großen Bogens 8 bis 25 mm, der Innenradius Rb des kleinen Bogens 4 bis 15 mm, wobei die beide Bögen verbindende Linie als Kurve mit einem Innenradius RL von 20 bis 150 mm ausgeführt ist. Die Wahl der Radien ist auf der einen Seite abhängig von den Gegebenheiten des Knochenbaues, also von der Größe des zu ersetzenden Gelenkes, sowie von der Menge der zu resezierenden Knochensubstanz, auf der anderen Seite aber auch abhängig vom eingesetzten Material der Femurkappe.

Grundsätzlich ist zunächst einmal davon auszugehen, daß so wenig Knochensubstanz abgetragen werden soll, wie sich vertreten läßt. Die Begründung für diese Forderung ist einfach darin zu sehen, daß man sich weitere Rückzugsmöglichkeiten für eine eventuelle Nachoperation zu einem wesentlich späteren Zeitpunkt offenhalten will, auf der anderen Seite aber auch, falls dann der Einsatz eines Gelenkes nicht mehr möglich ist, eine Gelenkversteifung vornehmen kann, ohne daß das Bein wesentlich kürzer wird. Die Verkürzung ergibt sich dabei zwangsläufig aus der Dicke der eingesetzten Implantatmaterialien, die gemäß einer bevorzugten Ausgestaltung der Erfindung bei der Femurkappe 4 mm nicht unter und 20 mm nicht überschreiten sollten. Die untere Grenze von 4 mm gestattet den sicheren Einsatz eines Metallimplantates, bei einer Femurkappe aus Oxidkeramik liegt dieser Wert höher, zweckmäßig nicht unter 6 mm. Die Obergrenze von 20 mm ist, wie bereits erwähnt, durch eine, bei der Versteifung des Gelenkes durch eine Nachoperation auftretende Verkürzung gegeben, die ebenfalls so gering wie möglich gehalten werden soll.

Beim Einsatz von Oxidkeramik, die wegen ihrer höheren Härte und damit der geringen Verschleißanfälligkeit bevorzugt wird, ist man bestrebt, die Radien nicht zu klein zu wählen, weil dadurch die Bearbeitungsvorgänge, wie Schleifen und Polieren, besser beherrschbar sind. Desweiteren ergibt sich beim Pressen der oxidkeramischen Pulver in ihre Form ein gleichmäßigerer Materialfluß und damit eine gleichmäßigere Verteilung der Festigkeit. Man ist also auch hier bestrebt, die untere Grenze des Innenradius des kleinen Bogens von 4 mm möglichst nicht auszuschöpfen, sondern darüber zu bleiben.

Das in die Femurkappe eingebrachte Langloch umfaßt gemäß einer zweckmäßigen Ausgestaltung der Erfindung einen Winkel von 90 bis 140 Grad. Der in dem Langloch geführte Schaft kann damit um mindestens 90 bis max. 140 Grad bewegt werden, d. h., daß die volle Bewegung des Kniegelenkes möglich ist.

Der Schaft ist dabei gemäß einer bevorzugten Ausgestaltung der Erfindung im Bereich des Langloches mit einer Führungshülse versehen. Die Führungshülse besteht dabei aus dem gleichen keramischen Material wie die Femurkappe und umgibt den metallischen Schaft im Bereich des Langloches, so daß bei der Bewegung des Gelenkes, also beim Beugen des Knies, Keramik auf Keramik gleitet und kein metallischer Abrieb entstehen kann.

Ist die Femurkappe als Metallimplantat ausgeführt, so besteht, um dem metallischen Abrieb vorzubeugen, der in ihr gleitende Zylinder aus HD-Polyäthylen, also einem Kunststoff. Die Führungshülse kann in diesem Falle nicht aus Oxidkeramik gefertigt werden, da das zum metallischen Abrieb im Bereich des Langloches der Femurkappe führen würde, sie wird also zweckmäßig ebenfalls aus dem gleichen Kunststoffmaterial gefertigt wie der Zylinder.

Der Schaft als solcher besteht aus den für Metallimplantate üblichen Werkstoffen, d. h. aus einer Legierung, die Chrom, Nickel, Molybdän und Kobalt enthalten kann, oder aber aus Titan. Er kann, wenn der Zylinder, in dem er befestigt werden soll, aus Hochdruckpolyäthylen besteht, durch eine « snap on » Verbindung mit diesem Zylinder verbunden werden. In diesem Fall weist der Schaft im Bereich des Eingriffs in den Zylinder eine Ringnut auf und der Zylinder im Bereich seiner Bohrung einen entsprechenden Ringwulst. Der Schaft kann dann durch Eindrücken in die Bohrung des Zylinders in diesem befestigt werden.

Eine bevorzugte Ausgestaltung der Erfindung sieht jedoch vor, daß der Zylinder eine konische Bohrung aufweist und der Schaft mit einem Konus zum Eingriff in die konische Bohrung des Zylinders versehen ist. Bei dieser Ausführungsform besteht der Zylinder aus einem gesinterten oxidkeramischen Material, das eine .konische Bohrung aufweist, während der Schaft mit einem Vaterkonus ausgestattet ist, der über eine verformbare Oberfläche verfügt. Durch das Zusammenwirken dieser beiden Aggregate tritt eine Selbsthemmung auf, wodurch der Schaft fest im Zylinder verankert wird.

Die Erfindung sieht ferner vor, daß der Femurkappe eine Tibiakappe zugeordnet ist. Ein direktes Einwirken der Femurkappe auf den Knorpel des Tibia-Plateaus führt zu einer punkt- und linienweisen Belastung des Knorpels und damit zu seiner Rückbildung. Das ist dadurch bedingt, daß man zwar versucht, die äußere Form der Femurkappe im wesentlichen der Form des natürlichen Knochenbaues anzupassen, eine absolute Anpassung aber nicht erreichen kann, weil von Patient zu Patient die Abmessungen der Condylen sich ändern und damit auch das Tibia-Plateau, das mit den Condylen in Eingriff steht, unterschiedlich aufgebaut ist. Die punkt- und/ oder linienförmige Belastung durch die Femurkappe, die zur Rückbildung des Knorpels führt, wirkt dann direkt auf das Tibia-Plateau ein, wobei auch hier eine Knochenrückbildung auftritt, d. h., daß der Zuordnung der Tibiakappe zur Femurkappe erhöhte Bedeutung zukommt.

Die Tibiakappe selbst weist gemäß einer bevorzugten Ausgestaltung der Erfindung C-förmigen Querschnitt auf und ist vorteilhafterweise im Bereich der Eminentia intercondylaris mit einer Durchgangsbohrung zur Aufnahme des Schaftes versehen. Als Material wird ein Kunststoff, vorzugsweise ein HD-Polyäthylen eingesetzt und zwar sowohl im Zusammenwirken mit einer Femurkappe aus Metall als auch im Zusammenwirken mit einer Femurkappe aus Oxidkeramik. In beiden Fällen ist die dämpfende Wirkung bei Stoßbewegungen sehr vorteilhaft, wesentlich ist aber auch, daß insbesondere bei der Kombination einer oxidkeramischen Femurkappe mit einer HDPE-Tibiakappe ausgezeichnete niedrige Reibungswerte erzielt werden.

Die Tibiakappe ist dabei in ihren äußeren Konturen weitgehend der natürlichen Form des Tibiaplateaus nachgebildet, weist also beidseitig der Eminentia intercondylaris je eine Pfanne zur Aufnahme der Femurcondylen auf. Durch ihren C-förmigen Querschnitt kann sie nach erfolgter Resektion des Tibiaplateaus auf die Tibia aufgeschoben werden, wobei auch hier eine Implantation ohne Knochenzement erfolgt. Die Primärfixation kann auch in diesem Fall durch Verschrauben oder Nageln erfolgen. Vorteilhafter ist jedoch das Einbringen des Schaftes in die Tibia durch eine Durchgangsbohrung in der Tibiakappe im Bereich der Eminentia intercondylaris.

Das Einbringen des Schaftes in die Tibia ist in jedem Fall erforderlich, wenn eine Femurkappe eingesetzt worden ist. Bei der zusätzlichen Implantation einer Tibiakappe kann also mit dem gleichen Schaft sowohl die Femurkappe als auch die Tibiakappe in ihrer Lage fixiert werden, was gegenüber dem Stand der Technik einen großen Fortschritt darstellt.

Die Erfindung wird nachstehend an Hand der Zeichnungen beschreiben.

Figur 1 zeigt als Explosionsschaubild in perspektivischer Darstellung eine Endoprothese,

Figur 2 zeigt die implantierte Endoprothese im Teilschnitt als Seitenansicht,

Figur 3 die implantierte Endoprothese als Rückansicht,

Figur 4 die gleiche als Vorderansicht,

Figur 5 einen Teilschnitt durch ein Kniegelenk,

Figur 6 die Rückansicht von Fig. 5 und

Figur 7 die Vorderansicht von zu Fig. 5,

Figur 8 eine alternative Lösung zu Fig. 5, bei der kein Zylinder in den Condylenbereich des Femur implantiert wurde,

Figur 9 den Stand der Technik.

Der Femur 15 ist im Bereich der Condylen 19 entlang der Oberflächen zur Aufnahme der Femurkappe 4 bearbeitet. Senkrecht zu dieser Oberflächenbearbeitung sind die Condylen 19 im gegeneinanderliegenden Bereich vertikal bearbeitet, d. h., daß sie im Bereich der Fossa intercondylaris 20 senkrecht verlaufende Flächen 21 aufweisen. Zwischen diese Flächen wird der Zylinder 10 eingelegt, der dann mit seinen Stirnflächen 12 die Flächen 21 der Condylen berührt. Die konische Bohrung 13 des Zylinders 10 ist dabei auf die Tibia 3 gerichtet. Die Mantelfläche 11 liegt auf der Fossa intercondylaris 20 auf.

Durch Aufpressen der C-förmigen Femurkappe 4 auf die resezierten Condylen 19 kommt der Zylinder 10 in den großen Bogen 5 der Femurkappe 4 zu liegen, deren Langloch 8 den Zugang zur konischen Bohrung 13 des Zylinders 10 freigibt. Der große Bogen 5 der Femurkappe 4 ist über die Linie 7 mit dem kleinen Bogen 6 verbunden, wobei die Linie 7 als schwachgekrümmter Bogen verläuft.

Das Plateau 26 der Tibia 3 ist zur Aufnahme der Tibiakappe 1 bearbeitet und weist ebenfalls einen großen und einen kleinen Bogen auf, wie er analog bei der Femurkappe 4 beschrieben wurde. Die Tibiakappe 1 ist mit einer Bohrung 2 im Bereich der Eminentia intercondylaris 16 versehen. Nach dem Aufbringen der Tibiakappe 1 auf die Tibia 3 erfolgt das Einbringen der Bohrung in die Tibia 3, woran sich das Eintreiben des Schaftes 9 anschließt. Der Schaft 9 ist mit Tragrippen 18 versehen, die eine sichere Befestigung in der Tibia 3 ermöglichen. Oberhalb der Tragrippen 18 weist er einen zylindrischen Bereich 22 auf, der zur Aufnahme der Führungshülse 17 dient. Daran schließt sich der Konus 14 an, der eine Verjüngung zwischen 1 : 10 und 1 : 20 aufweist und in die konische Bohrung 13 des Zylinders 10 eingreift. Die Tibiakappe 1 ist dabei so ausgeführt, daß die Fibula 23 im Normalfall nicht bearbeitet werden muß.

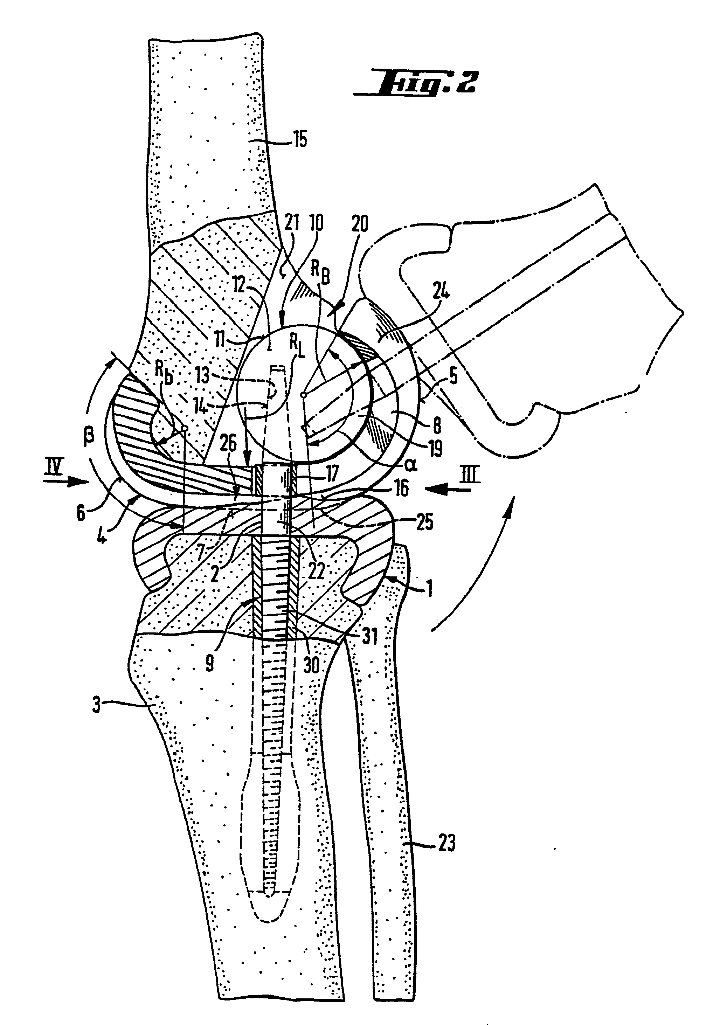

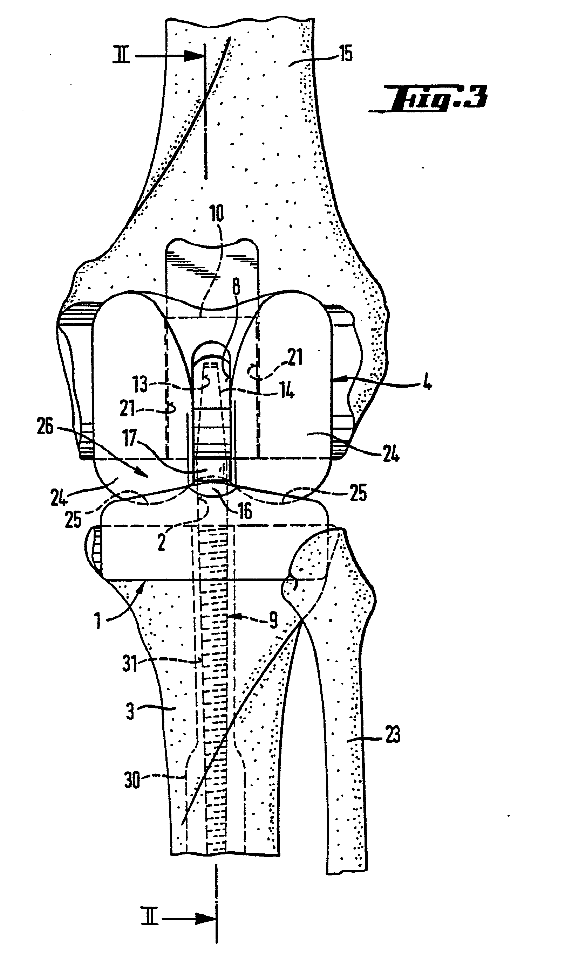

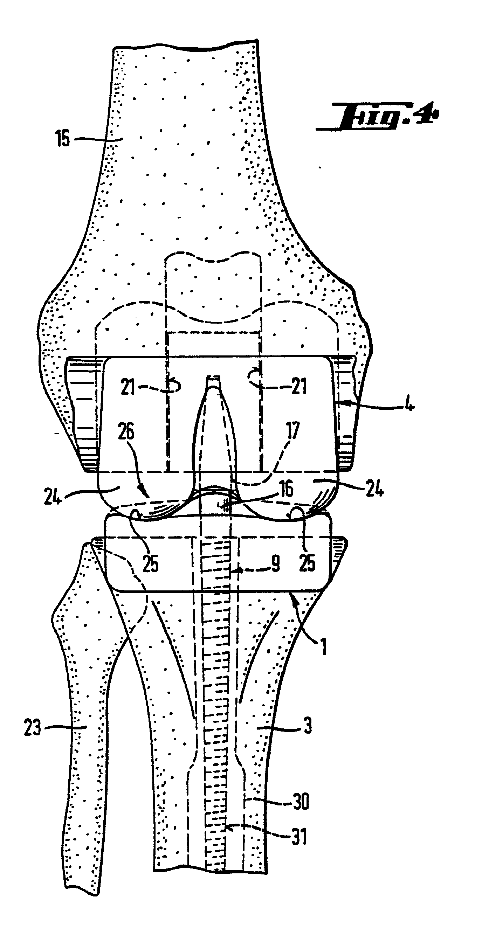

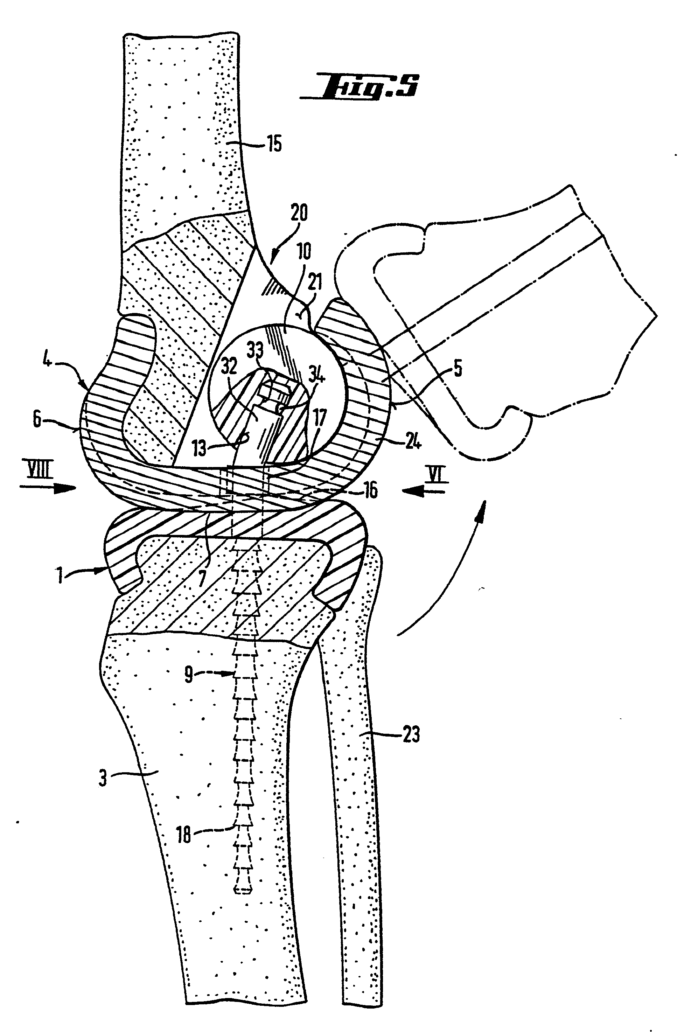

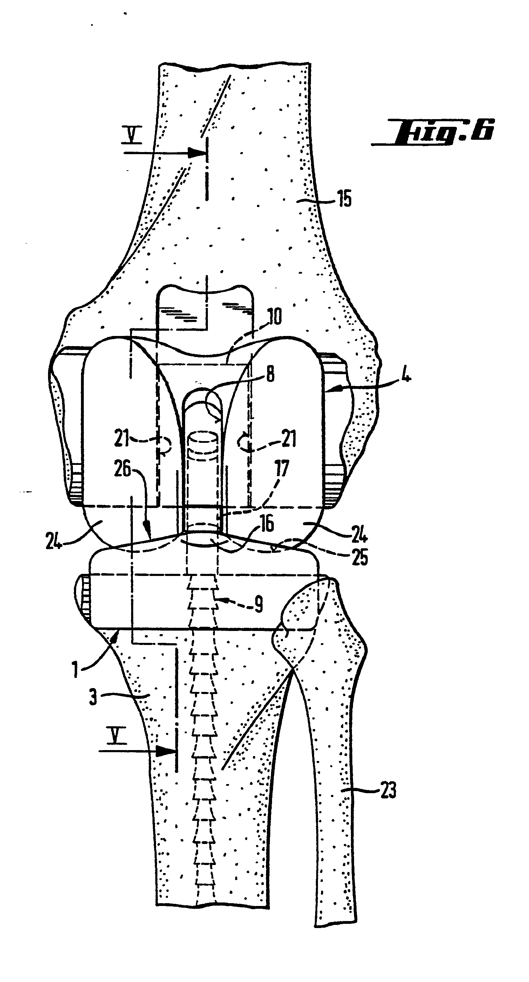

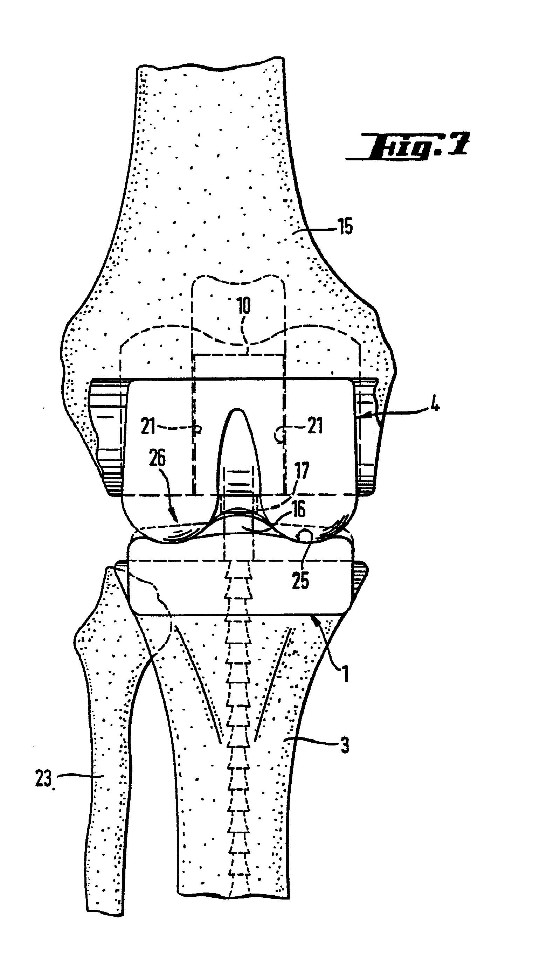

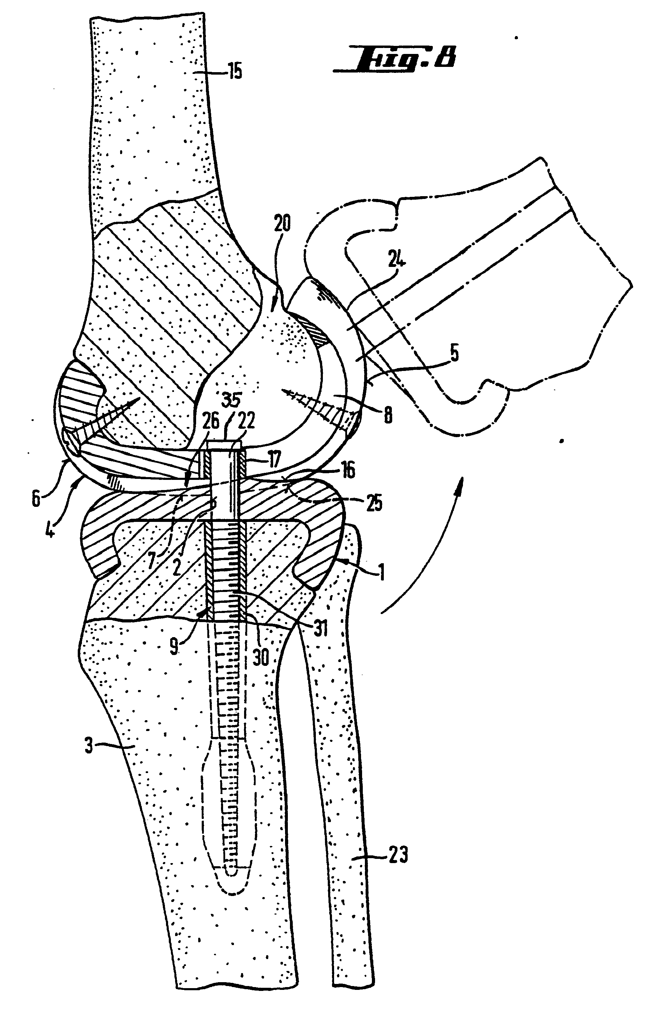

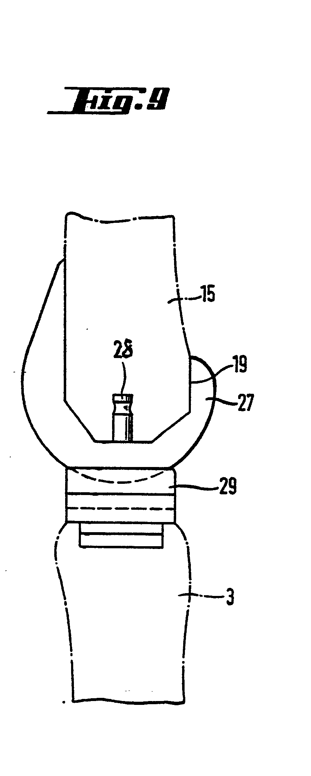

Die Figuren 2, 3, 4 und 8 zeigen eine andere Ausführungsform des Schaftes 9. Der Schaft 9 ist in diesen Fällen mit einem Gewindeeinsatz 31 versehen, der in einen Spreizdübel 30 eingebracht wird, so daß durch Eindrehen des Gewindeeinsatzes 31 der Spreizdübel 30 an die Wandung der Tibia 3 angedrückt und damit die Tibiakappe 1 in ihrer Position auf der Tibia 3 fixiert wird.

Gemäß Fig. 5 ist der Schaft 9 in seinem oberen Bereich abgekröpft und mit einem Zylinderzapfen 32 versehen. Dieser Zylinderzapfen 32 weist eine Ringnut 33 auf, in die ein Ringwulst 34, der in der Bohrung 13 des Zylinders 10 angeordnet ist, eingreift. In diesem Fall besteht der Zylinder 10 aus HD-Polyäthylen, d. h., daß der Zylinderzapfen 32, der in seinem vorderen Bereich konisch angefast ist, in die Bohrung 13 eingetrieben werden kann, hier den Ringwulst 34 auf Grund der Elastizität des Materials auseinanderdrückt, wobei dann dieser Ringwulst 34 im Anschluß daran zurückfedert und in die Ringnut 33 eingreift. Der Schaft 9 ist dadurch fest im Zylinder 10 verankert.

Bei gestecktem Bein verläuft die Linie 7 der Femurkappe 4 praktisch in der Horizontalen, d. h. senkrecht zur Achse des Femur 15. Die Condylennachbildung 24 der Femurkappe 4, ein ungefähr tonnenförmiger Bereich, ruht dabei in den komplementär ausgestalteten Pfannen 25 der Tibiakappe 1 — Figuren 2 bis 4 —.

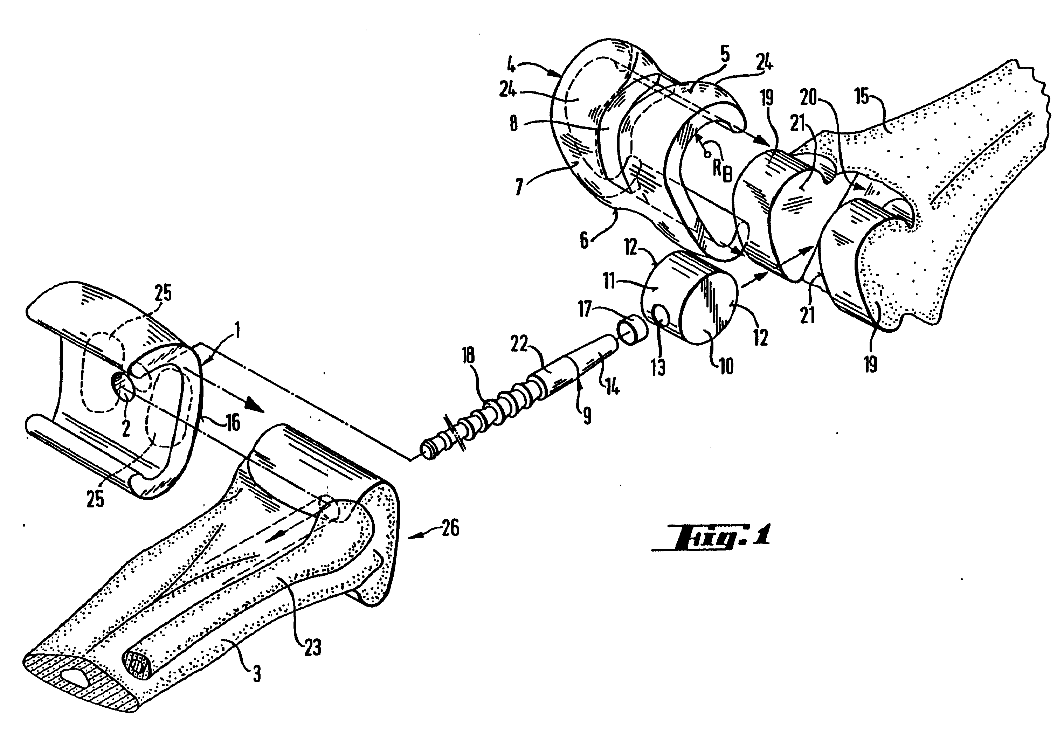

Die Figuren 2 bis 8 zeigen eine implantierte Femurkappe 4 im direkten Eingriff mit der Tibiakappe 1. Bei den Figuren 2 bis 7 ist analog der Figur 1 der Eingriff des Schaftes 9 in den Zylinder 10 dargestellt, was zwar äußerst vorteilhaft, aber nicht bei jeder Operation erforderlich ist. Figur 8 zeigt daher die Fixation der Femurkappe 4 durch eine Schraubbefestigung, wobei in diesem Falle keine Resektion im Bereich der Fossa intercondylaris 20 erforderlich war. Daher ist der Schaft 9, der weiterhin zur Führung erdorderlich ist, kürzer ausgeführt, d. h., er ragt in das Langloch 8 der Femurkappe 4 hinein, greift aber nicht wesentlich durch dieses in den Bereich der Fossa intercondylaris 20 hinein. Auch in diesem Fall ist der Schaft 9 wieder mit einer Führungshülse 17 versehen, die auf ihm durch den Kopf 35 des Schaftes 9 gehalten wird.

Die Fig. 5 zeigt ferner oberhalb des kleinen Bogens 6 der Femurkappe 4 einen hochgezogenen Bereich, der dann erforderlich ist, wenn auch die Patella ersetzt werden mußte, wobei dieser Bereich als Führung für die künstliche Patella gilt.

Die Fig. 9 zeigt den Stand der Technik, bei dem der Femur 15 im Bereich der Condylen 19 wesentlich stärker reseziert ist und von einem hakenförmigen Implantat 27 umgriffen wird. Dieses hakenförmige Implantat 27 ist mittels Kitt und eines Haltestiftes 28 am Femur 15 befestigt und ruht in einer ebenfalls mit Kitt in die Tibia 3 eingesetzten Lagerplatte 29.

**Patentansprüche**

1. Kniegelenkendoprothese zur Verbindung des Femur (15) mit der Tibia (3), bestehend aus einer Femurkappe (4) mit hakenförmigem Querschnitt zur teilweisen Umfassung der resezierten Femur-Condylen (19) und gleichzeitiger Abstützung und Lagerung auf dem Tibia-Plateau (26) sowie einem in die Tibia implantierbaren Verbindungsstück (9), gekennzeichnet durch die Kombination folgender Merkmale :

a) der Querschnitt der Femurkappe (4) ist C-förmig,

b) der C-förmige Querschnitt der Femurkappe (4) ist durch zwei unterschiedlich große Bögen (5, 6) gebildet, die durch eine Linie (7) miteinander verbunden sind,

c) im Bereich des großen Bogens (5) der Femurkappe (4) verläuft radial ein Langloch (8), durch das sich ein in die Tibia (3) implantierbarer Schaft (9) erstreckt.

2. Kniegelenkendoprothese nach Anspruch 1, dadurch gekennzeichnet, daß der Schaft (9) in einem, im Bereich des großen Bogens (5) drehbar angeordneten, sich beidseitig über das Langloch (8) hinaus erstreckenden Zylinder (10) befestigt ist.

3. Kniegelenkendoprothese nach einem der Ansprüche 1 und 2, dadurch gekennzeichnet, daß die Femurkappe (4) in dem an das Langloch (8) angrenzenden Innenbereich des großen Bogens (5) poliert ist.

4. Kniegelenkendoprothese nach einem der Ansprüche 1 bis 3, dadurch gekennzeichnet, daß der Umfang der Femurkappe (4) poliert ist.

5. Kniegelenkendoprothese nach einem der Ansprüche 1 bis 4, dadurch gekennzeichnet, daß der große Bogen (5) einen Winkel $\alpha$ von 130 bis 190 Grad umfaßt.

6. Kniegelenkendoprothese nach einem der Ansprüche 1 bis 5, dadurch gekennzeichnet, daß der kleine Bogen (6) einen Winkel $\beta$ von 120 bis 160 Grad umfaßt.

7. Kniegelenkendoprothese nach einem der Ansprüche 1 bis 6, dadurch gekennzeichnet, daß die Linie (7) als Kurve mit einem Innenradius $R_L$ von 20 bis 150 mm ausgeführt ist.

8. Kniegelenkendoprothese nach einem der Ansprüche 1 bis 7, dadurch gekennzeichnet, daß die Wandstärke der Femurkappe (4) 4 bis 20 mm beträgt.

9. Kniegelenkendoprothese nach einem der Ansprüche 1 bis 8, dadurch gekennzeichnet, daß der Innenradius $R_B$ des großen Bogens (5) 8 bis 25 mm beträgt.

10. Kniegelenkendoprothese nach einem der Ansprüche 1 bis 9, dadurch gekennzeichnet, daß der Innenradius $R_b$ des kleinen Bogens (6) 4 bis 15 mm beträgt.

11. Kniegelenkendoprothese nach einem der Ansprüche 1 bis 10, dadurch gekennzeichnet, daß das Langloch (8) einen Winkel von 90 bis 140 Grad umfaßt.

12. Kniegelenkendoprothese nach einem der Ansprüche 1 bis 11, dadurch gekennzeichnet, daß der Schaft (9) im Bereich des Langloches (8) mit einer Führungshülse (17) versehen ist.

13. Kniegelenkendoprothese nach einem der Ansprüche 1 bis 12, dadurch gekennzeichnet, daß die Mantelfläche (11) des Zylinders (10) poliert ist.

14. Kniegelenkendoprothese nach einem der Ansprüche 1 bis 13, dadurch gekennzeichnet, daß die Stirnflächen (12) des Zylinders (10) poliert sind.

15. Kniegelenkendoprothese nach einem der Ansprüche 1 bis 14, dadurch gekennzeichnet, daß der Zylinder (10) eine konische Bohrung (13) aufweist und der Schaft (9) mit einem Konus (14) zum Eingriff in die konische Bohrung (13) des Zylinders (10) versehen ist.

16. Kniegelenkendoprothese nach einem der Ansprüche 1 bis 15, dadurch gekennzeichnet, daß der Femurkappe (4) eine Tibiakappe (1) zugeordnet ist.

17. Kniegelenkendoprothese nach einem der Ansprüche 1 bis 16, dadurch gekennzeichnet, daß die Tibiakappe (1) C-förmigen Querschnitt aufweist.

18. Kniegelenkendoprothese nach einem der Ansprüche 1 bis 17, dadurch gekennzeichnet, daß die Tibiakappe (1) im Bereich der Eminentia intercondylaris (16) eine Durchgangsbohrung (2) zur Aufnahme des Schaftes (9) aufweist.

**Claims**

1. Knee-joint endoprosthesis for connecting the femur (15) to the tibia (3), comprising a femur cap (4) having a hook-shaped cross-section for partial enclosure of the resected femur condyles (19) and simultaneous support and location on the tibia plateau (26), and also a connecting piece (9) that can be implanted in the tibia, characterised by the combination of the following features :

a) the cross-section of the femur cap (4) is C-shaped ;

b) the C-shaped cross-section of the femur cap (4) is formed by two curved portions (5, 6) of different sizes which are joined to one another by a line (7) ;

c) in the region of the large curved portion (5) of the femur cap (4) there extends radially a slot (8) through which extends a shaft (9) that can be implanted in the tibia (3).

2. Knee-joint endoprosthesis according to claim 1, characterised in that the shaft (9) is fastened in a cylinder (10) which is arranged in the region of the large curved portion (5) in such a manner that it can be rotated and which extends beyond both sides of the slot (8).

3. Knee-joint endoprosthesis according to one of claims 1 and 2, characterised in that the femur cap (4) is polished in the inner region of the large curved portion (5), adjacent to the slot (8).

4. Knee-joint endoprosthesis according to one of claims 1 to 3, characterised in that the periphery of the femur cap (4) is polished.

5. Knee-joint endoprosthesis according to one of claims 1 to 4, characterised in that the large curved portion (5) forms an angle $\alpha$ of from 130 to 190 degrees.

6. Knee-joint endoprosthesis according to one of claims 1 to 5, characterised in that the small curved portion (6) forms an angle $\beta$ of from 120 to 160 degrees.

7. Knee-joint endoprosthesis according to one of claims 1 to 6, characterised in that the line (7) is in the form of a curve having an internal radius $R_L$ of from 20 to 150 mm.

8. Knee-joint endoprosthesis according to one of claims 1 to 7, characterised in that the wall thickness of the femur cap (4) is from 4 to 20 mm.

9. Knee-joint endoprosthesis according to one of claims 1 to 8, characterised in that the internal radius $R_B$ of the large curved portion (5) is from 8 to 25 mm.

10. Knee-joint endoprosthesis according to one of claims 1 to 9, characterised in that the internal radius $R_b$ of the small curved portion (6) is from 4 to 15 mm.

11. Knee-joint endoprosthesis according to one of claims 1 to 10, characterised in that the slot (8) forms an angle of from 90 to 140 degrees.

12. Knee-joint endoprosthesis according to one of claims 1 to 11, characterised in that, in the region of the slot (8), the shaft (9) is provided with a guide sleeve (17).

13. Knee-joint endoprosthesis according to one of claims 1 to 12, characterised in that the convex surface (11) of the cylinder (10) is polished.

14. Knee-joint endoprosthesis according to one of claims 1 to 13, characterised in that the end faces (12) of the cylinder (10) are polished.

15. Knee-joint endoprosthesis according to one of claims 1 to 14, characterised in that the cylinder (10) has a conical bore (13) and the shaft (9) is provided with a tapered portion (14) for engaging in the conical bore (13) of the cylinder (10).

16. Knee-joint endoprosthesis according to one of claims 1 to 15, characterised in that a tibia cap (1) is associated with the femur cap (4).

17. Knee-joint endoprosthesis according to one of claims 1 to 16, characterised in that the tibia cap (1) has a C-shaped cross-section.

18. Knee-joint endoprosthesis according to one of claims 1 to 17, characterised in that, in the region of the Eminentia intercondylaris (16), the tibia cap (1) has a through-bore (2) for accommodating the shaft (9).

**Revendications**

1. Endoprothèse du genou pour le raccordement du fémur (15) au tibia (3), comprenant une coiffe de fémur (4) à section transversale en forme de crochet pour l'enveloppement partiel des condyles préparés (19) du fémur et le montage et la fixation simultanés sur le plateau tibial (26), ainsi qu'un élément de liaison (9) pouvant être implanté dans le tibia, caractérisé par la combinaison des caractéristiques suivantes :

a) la section transversale de la coiffe de fémur (4) est en forme de C,

b) la section transversale en forme de C de la coiffe de fémur (4) est formée par deux arcs (5, 6) de grandeur différente et mutuellement raccordés par une ligne (7),

c) dans la zone du grand arc (5) de la coiffe de fémur (4) est disposé radialement un trou oblong (8) à travers lequel s'étend une tige (9) pouvant être implantée dans le tibia (3).

2. Endoprothèse du genou selon la revendication 1, caractérisée par le fait que la tige (9) est fixée dans un cylindre (10) monté rotatif dans la zone du grand arc (5) et débordant des deux côtés du trou oblong (8).

3. Endoprothèse du genou selon l'une des revendications 1 et 2, caractérisée par le fait que la coiffe de fémur (4) est polie dans la zone interne du grand arc (5) voisine du trou oblong (8).

4. Endoprothèse du genou selon l'une des revendications 1 à 3, caractérisée par le fait que la périphérie de la coiffe de fémur (4) est polie.

5. Endoprothèse du genou selon l'une des revendications 1 à 4, caractérisée par le fait que le grand arc (5) englobe un angle $\alpha$ de 130 à 190°.

6. Endoprothèse du genou selon l'une des revendications 1 à 5, caractérisée par le fait que le petit arc (6) englobe un angle $\beta$ de 120 à 160°.

7. Endoprothèse du genou selon l'une des revendications 1 à 6, caractérisée par le fait que la ligne (7) est réalisée sous la forme d'une courbe ayant un rayon intérieur ($R_L$) de 20 à 150 mm.

8. Endoprothèse du genou selon l'une des revendications 1 à 7, caractérisée par le fait que l'épaisseur de la paroi de la coiffe de fémur (4) est de 4 à 20 mm.

9. Endoprothèse du genou selon l'une des revendications 1 à 8, caractérisée par le fait que le rayon intérieur ($R_B$) du grand arc (5) est de 8 à 25 mm.

10. Endoprothèse du genou selon l'une des revendications 1 à 9, caractérisée par le fait que le rayon intérieur ($R_b$) du petit arc (6) est de 4 à 15 mm.

11. Endoprothèse du genou selon l'une des revendications 1 à 10, caractérisée par le fait que le trou oblong (8) couvre un angle de 90 à 140°.

12. Endoprothèse du genou selon l'une des revendications 1 à 11, caractérisée par le fait que la tige (9) est munie d'une douille de guidage (17) dans la zone du trou oblong (8).

13. Endoprothèse du genou selon l'une des revendications 1 à 12, caractérisée par le fait que la surface latérale (11) du cylindre (10) est polie.

14. Endoprothèse du genou selon l'une des revendications 1 à 13, caractérisée par le fait que les surfaces frontales (12) du cylindre (10) sont polies.

15. Endoprothèse du genou selon l'une des revendications 1 à 14, caractérisée par le fait que le cylindre (10) comporte un perçage conique (13) et que la tige (9) est munie d'un cône (14) s'engageant dans le perçage conique (13) du cylindre (10).

16. Endoprothèse du genou selon l'une des revendications 1 à 15, caractérisée par le fait qu'à la coiffe de fémur (4) est associée une coiffe de tibia (1).

17. Endoprothèse du genou selon l'une des revendications 1 à 16, caractérisée par le fait que la coiffe de tibia (1) a une section transversale en forme de C.

18. Endoprothèse du genou selon l'une des revendications 1 à 17, caractérisée par le fait que la coiffe de tibia (1) dans la zone de l'épine de tibia (16) comporte un perçage (2) permettant le passage de la tige (9).

Fig.1

Fig.2

*Fig.3*

**Fig.4**

Fig.5

Fig.6

Fig.1

Fig. 8

# Fig.9